# EUROPEAN PATENT APPLICATION

(11) **EP 1 704 879 A1**
(43) Date of publication of application: **27.09.2006**
(21) Application number: 05006489.8
(22) Date of filing: 24.03.2005
(51) Int. Cl.: A61L 29/16, A61L 29/14, A61L 33/00

(54) **Intravascular, interstitial or intraorgan medical device comprising a nitric oxide eluting polymer**

(71) Applicant: NOLabs AB, 252 21 Helsingborg (SE)
(72) Inventor: Peters, Tor, 8703 Erlenbach (CH)
(74) Representative: Petri, Stellan

(57) **Abstract**

A intravascular, interstitial or intraorgan medical device, and a manufacturing process of said medical device, is provided that allows for prevention of infection and obtainment of anti-thrombotic effect. The medical device comprises a nitric oxide (NO) eluting polymer arranged adjacent mammal tissue, such that a therapeutic dose of nitric oxide is eluted from said nitric oxide eluting polymer to said mammal tissue.

## Description

### Field of the Invention

This invention pertains in general to the field of a medical device, involving the use of nitric oxide (NO). More particularly the invention relates to an intravascular, interstitial or intraorgan medical access device, and a process for manufacturing of said device, involving the use of nitric oxide (NO).

### Background of the Invention

In the field of medicine a lot of medical devices are inserted, implanted, or attached to the body of a mammal, such as a human. These devices are intended to fulfil different medical purposes, such as closing a wound or operation wound, draining different kinds of body fluids from for example intraorgan, such as the pleura, urinary bladder, inner ear, or the vascualtory system system, etc., for isntance by the aid of catheters, and injecting medicaments, drugs, saline etc. Some medical devices are simply in contact with the mammal body, such as colostomy bags etc, or intended for supervision of blood pressure, blood gases etc. When these medical devices are inserted, implanted, or attached to the mammal body, the risk of infection is seriously increased.

Catheters are flexible rubber, or plastic, tubes that are inserted into different parts of the body, to provide a channel for fluid passage or another medical device. A catheter may for example remove waste fluids from the body after transurethral resection, or surgical operations in the lung.

A urinary catheter, such as a Foley catheter or balloon catheter, is inserted into the urinary bladder to drain urine. Because it can be left in place in the urinary bladder for a period of time, it is also called an indwelling catheter. It is held in place with a balloon in the end, which is filled with sterile water, or air, in order to hold the catheter in place. Since the catheter is in place for a period of time, a number of side effects may occur, such as infections from bacteria, fewer, urosepsis etc. The bacteria present in, and in the vicinity of, the catheter may also be stone forming bacteria, which may result in blockage of the catheter. These disorders are today treated with antibiotics, but it is today common knowledge that treatment with antibiotics may result in development of bacteriological resistance against antibiotics, which may lead to severe complications in case of infections.

An intravenous catheter is inserted in a venous blood vessel to facilitate repeated injections, infusions, transfusions and blood samplings, and include central vein catheters (CVC), peripheral vein catheters (PVC), and subcutaneous vein ports (SVP). Also this type of catheter may cause infection, which infection today is treated with antibiotics entailing the side effects according to above. However, it is important to suppress this infections in order not to lead to local and systemic infectious complications, including local site infection, septic thrombophlebitis, endocarditis, and other metastatic infections, e.g., lung abscess, brain abscess, osteomyelitis, and endophthalmitis.

Another problem that may arise in the intravenous catheters according to the prior art is blockage of the catheters, due to coagulation of blood present in the catheters. Therefore, the catheters according to prior art have to be flushed with saline before and after each injection, infusion, transfusion and blood sampling, to ensure faultless infusion or injection.

In respect of closing wounds or operation wounds, sutures and staples are the most commonly used medical devices, in respect of both internal and external wounds. These staples and sutures are to keep the margins of the skin or tissue closed. These staples or sutures must be removed within 14 days from application. Otherwise they may cause complications, in form of infections, which infections today are treated with antibiotics entailing the side effects according to above.

Nitric oxide (NO) is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without having adverse effects.

However, due to the short half-life of NO, it has hitherto been very hard to treat viral, bacteria, virus, fungi or yeast infections with NO. This is because NO is actually toxic in high concentrations and has negative effects when applied in too large amounts to the body.

NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible with natural products, after the release of nitrogen oxide.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices to be permanently implanted in the body, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning an improvement of present technology in respect of medical devices for preventing infection and obtaining anti-thrombotic effect by the use of NO.

Hence, an improved intravascular, interstitial or intraorgan medical access device, and a manufacturing process therefor, for preventing infection, which device presents an wound healing promoting and anti-infectious, anti-microbial, anti-inflammatory, anti-thrombotic, and/or anti-viral effect, would be advantageous.

### Sununary of the Invention

Accordingly, the present invention preferably seeks to mitigate, alleviate or eliminate one or more of the above-identified deficiencies in the art and disadvantages singly or in any combination and solves at least the problems mentioned above, by providing a medical device, for preventing infection and obtaining anti-thrombotic effect, a manufacturing method for the latter and a use of nitric oxide according to the appended patent claims.

According to one aspect of the invention, a medical device is provided that allows for prevention of infection and obtainment of anti-thrombotic effect. The device comprises a nitric oxide (NO) eluting polymer adjacent to an area of mammal tissue or body fluid, such that a therapeutic dose of nitric oxide is eluted from said nitric oxide eluting polymer to said area.

According to another aspect of the invention, a manufacturing process for such a medical device is provided, wherein the process is a process for forming a device that allows for prevention of infection and obtainment of anti-thrombotic effect. The process comprises selecting a plurality of nitric oxide eluting polymeric particles, such as nano fibres, fibres, nano particles, or microspeheres, and deploying said nitric oxide eluting particles in, or on, said medical device.

The present invention has at least the advantage over the prior art that it provides a medical device with target exposure to NO, whereby prevention of infection and obtainment of anti-thrombotic effect, simultaneously as an anti-viral, an anti-inflammatory, and an anti-microbial therapy, are achievable.

### Brief Description of the Drawings

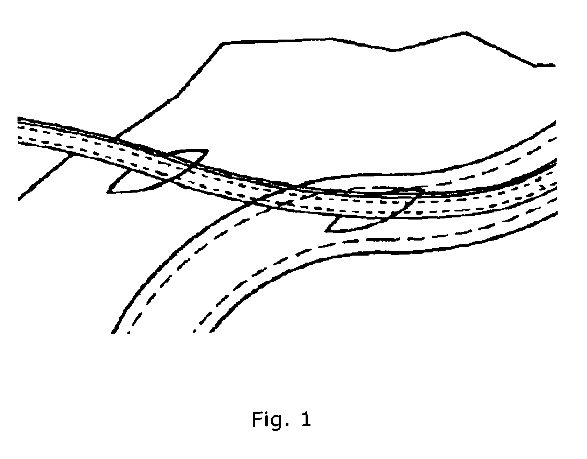

These and other aspects, features and advantages of which the invention is capable of will be apparent and elucidated from the following description of embodiments of the present invention, reference being made to the accompanying drawings, in which
Fig. 1 is a schematic illustration of a catheter according to the invention.

### Description of Embodiments

The following description focuses on embodiments of the present invention applicable to a intravascular, interstitial or intraorgan medical device, which device allows for target exposure to NO, whereby prevention of infection and obtainment of anti-thrombotic effect is achieved, simultaneously as an anti-viral, an anti-inflammatory, and an anti-microbial therapy may be provided.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, anti-platelet-aggregating action, anti-bacterial action, anti-viral action, anti-inflammatory action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide. Another advantage is that NO is released without any secondary products that could lead to undesired side effects.

The polymers according to the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

In one embodiment of the device according to the present invention, said device is in form of a catheter, according to Fig. 1, which catheter is suitable to be used for draining different kinds of body fluids from for example pleura, urinary bladder, blood system, ear, etc., by the aid of catheters, and injecting medicaments, drugs, saline etc.

The core material of the catheter according to the present invention may be any suitable material according to the prior art, such as silicone, latex, polytetraflouroethene, polyurethane, polyvinylchloride, polycarbonate, Acrylonitrile Butadiene Styrene (ABS), polyacrylate, polyamide, polyethene, polypropene, polyolefins, polystyrene, rubbers, and/or any combinations of these.

The surface of said core material is then covered with the NO eluting polymers according to the present invention. This is accomplished by electro spinning, air spinning, or nano-spinning of said NO eluting polymer onto said core material.

In another embodiment of the present invention the NO eluting polymer according to the invention is integrated in the core material. This embodiment has the advantage of easier presentation of NO eluting polymer on the surface of the catheter facing the body fluid, such as blood, to thereby obtaining an anti-thrombotic effect on this side of the catheter.

This may be accomplished by integrating fibres, nano-particles or micro-spheres of the NO-eluting polymer according to the present invention in the core material prior to the moulding of said catheter.

These fibres, nano-particles, or micro-spheres, may be formed from the NO-eluting polymers comprised in the present invention, for example polyalkyleneimines, such as L-PEI (Linear PolyEthyleneImine) and B-PEI (Branched PolyEthyleneImine), which polymers have the advantage of being biocompatible, after the release of nitrogen oxide.

They may also be encapsulated in any suitable material, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics. This encapsulation is performed if, by any reason, the elution of NO needs to be regulated in respect of time.

The device according to the present invention is applied on the intended area, such as urethra, bloodstream, pleura, pharynx, trachea, etc.

When the device according to the present invention has been applied, an elution of NO is initiated when the device, including the NO eluting polymer according to the present invention, gets in contact with the moisture or water in the adjacent tissue of the mammal body.

A therapeutic effect of the application area is obtained, as the NO eluting polymer elutes NO on the application area, whereby an anti-microbial, anti-inflammatory, anti-thrombotic or anti-viral effect of the tissue of interest is achieved.

The increased blood perfusion and vasodilatation may, in another embodiment of the present invention, result in an improved effect when combined with other active components. Thus, the synergistic effect from NO and other active components is within the scope of the present invention.

In other embodiments of the present invention the device according to the present invention may be selected from the group consisting of venflones; catheters, such as urinary catheters, central vein catheters (CVC), peripheral vein catheters (PVC), and subcutaneous vein ports (SVP); drainage tubes, such as tubes for pleura drainage and other wound drainages; articles intended for supervision of pressure and/or blood gases; and intravenous dressings.

When the device according to the present invention is in form of a urinary catheter, said urinary catheter is provided with anti-microbial, anti-inflammatory, anti-thrombotic and anti-viral effect. Thereby, the urinary catheter may by in place for a long period of time, while still conquering the majority of the side effects according to the prior art, such as infections from bacteria, fewer, urosepsis, and/or bacteriological stone formation. Furthermore, there is hence no need for treatment with antibiotics.

When the device according to the present invention is in form of a venflone, a central vein catheter, a peripheral vein catheter, and a subcutaneous vein port, said devices are provided with anti-microbial, anti-inflammatory, anti-thrombotic and anti-viral effect. Thereby, said devices may be in place for a long period of time, while still conquering the majority of side effects according to the prior art, such as infections from bacteria, and blockage of the devices, due to coagulation of blood present in the catheters. Therefore, there is no need for flushing the devices according to the invention prior to and after each injection, infusion, transfusion and blood sampling, to ensure faultless infusion or injection.

When the device according to the present invention is in form of a drainage tube, said drainage tube is provided with anti-microbial, anti-inflammatory, anti-thrombotic and anti-viral effect. Thereby, said devices may be in place for a long period of time, while still conquering the majority of side effects according to the prior art, such as infections from bacteria, and blockage of the drainage tube, due to coagulation of blood present in the drainage tube.

When the device according to the present invention is in form of an article for the supervision of blood pressure or blood gases, said device is provided with anti-microbial, anti-inflammatory, anti-thrombotic and anti-viral effect. Thereby, said device may be in place for a long period of time, while still conquering the majority of side effects according to the prior art, such as infections from bacteria, and blockage of the devices, due to coagulation of blood present in the catheters.

In another embodiment of the device according to the present invention is in form of sutures or staples. The sutures and staples according to the invention are provided with anti-microbial, anti-inflammatory, and anti-viral effect. Thereby, said sutures and staples may be in place for a long period of time, while still conquering the majority of side effects according to the prior art, such as infections from bacteria. Therefore, the staples or sutures according to the present invention need not be removed within 14 days from application, which is the case with the sutures and staples according to the prior art. Since the sutures and staples according to the invention provides an anti-inflammatory effect, the risk of need for treatment with antibiotics is significantly reduced.

When the device according to the present invention is in form of a intravenous dressing, the area surrounding an intravenous catheter is provided with anti-microbial, anti-inflammatory, anti-thrombotic and anti-viral effect. This embodiment has the advantage of protecting an area which otherwise is exposed to a high possibility of getting in contact with infectious material.

The NO-eluting polymer may be integrated in, spun together with, or spun on top of, any of these devices in all of the embodiments of the present invention.

Preferably the aforementioned embodiments employ L-PEI material loaded with NO. Activation on NO release will be achieved when the devices according to all the embodiments of the present invention get in contact with the moisture and/or water of the adjacent tissue of the mammal.

In another embodiment the fibres, nano-particles, or micro-spheres, may be integrated in a soluble film that disintegrates on the inside of the devices according to the present invention, in order to elute NO at the area of interest when the soluble film gets in contact with the moisture or water in the adjacent tissue of the mammal.

In another embodiment of the present invention the device according to the present invention only allows NO-elution in one direction. In this kind of embodiment one side of the device according to the invention is non-permeable to NO. This may be accomplished by applying a material on one side of the device according to the invention that is not permeable to NO. Such materials may be chosen from the group comprising common plastics, such as polyethylene, polyurethane etc. This embodiment is also easy to manufacture as the NO eluting polymer, e.g. L-PEI nano fibres may be electro or gas-jet spun onto the surface of the device according to the invention of e.g. the mentioned plastics, latex, or cotton. This may protect the NO eluting polymer during packaging, transport and prior to use from external influences, being e.g. mechanical (abrasion of the polymer), chemical (moisture deactivating the device prior to use) etc.

In yet another embodiment of the present invention the NO-eluting device is acting as a booster for medications and pharmaceuticals. This embodiment presents a device with the advantage of combining two treatments, of significant value, in one treatment.

Hence, such devices may achieve a synergetic effect, when NO is eluted from the devices. NO has a vasodilatory effect. Vasodilated tissue is more susceptible to certain medications and pharmaceuticals, and thus more easily treated by the medical preparations and still NO has the anti-inflammatory, anti-bacterial, anti-thrombotic, and anti-viral effect. Hence, an unexpected surprisingly effective treatment is provided.

Catheters are normally manufactured by extrusion. When manufacturing catheters and venflones according to the present invention, the NO eluting polymer may be integrated in the polymer blend that will be extruded. This manufacturing process provides catheters and venflones with the ability to elute NO to the fluid, or body fluid, passing through said catheters/venflones.

In another manufacturing process according to the present invention the catheters and venflones are manufactured in a two step process. In the first step the catheters/venflones are extruded. In the second step the catheters/venflones are covered on the in- and outside with NO eluting polymer by electro-spinning, nano-spinning or air-spinning.

The device according to the present invention elutes nitric oxide (NO) from said eluting polymer in a therapeutic dose in the oral cavity, such as between 1 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

The NO-eluting polymers in the devices according to the present invention may be combined with silver, such as hydroactivated silver. The integration of silver in the devices according to the present invention gives the therapeutic treatment an extra boost. Preferably the silver is releasable from the devices in the form of silver ions.

The device according to the present invention may be manufactured by, for example electro spinning of L-PEI or other polymers comprising L-PEI or being arranged in combination with L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the device according to the invention.

It is of course possible to electro spin the other NO-eluting polymers, according to above, on the device according to the invention while still being inside the scope of the present invention.

In one embodiment the NO-eluting polymers according to the present invention are electro spun in such way that pure NO-eluting polymer fibres may be obtained.

It is also within the scope of the present invention to electro spin a NO-eluting polymer together with other suitable polymer/polymers.

Gas stream spinning, or air spinning, of said NO-eluting polymers onto the device according to the present invention is also within the scope of the present invention.

The manufacturing process according to the present invention presents the advantages of large contact surface of the NO-eluting polymer fibres with the area to be treated, effective use of NO-eluting polymer, and a cost effective way of producing the device according to the present invention.

Hereinafter an example of a use of the present invention will be disclosed:

A therapeutic method for prevention of infection and/or obtainment of anti-thrombotic effect when using a intravascular, interstitial or intraorgan medical device, comprising deploying a intravascular, interstitial or intraorgan medical device, as described above, to a site entering a mammal body, and exposing an adjacent area of mammal tissue to nitric oxide eluted from a polymer of said device during use thereof.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. An intravascular, interstitial or intraorgan medical access device that allows for prevention of infection and/or obtainment of anti-thrombotic effect,
wherein
said device comprises a nitric oxide (NO) eluting polymer configured for eluting a therapeutic dosage of nitrogen oxide (NO), and
wherein said device is configured for exposing an adjacent area of mammal tissue to said nitric oxide when said polymer in use elutes nitric oxide (NO).

2. Medical device according to claim 1, wherein said polymer is selected from the group consisting of polyalkyleneimines, S-nitrosylated polymer, and poly(alkylenimine)diazeniumdiolates, or any combinations thereof.

3. Medical device according to claim 1, wherein said polymer is L-PEI (linear polyethyleneimine), loaded with nitric oxide (NO), arranged for release of the nitric oxide (NO) to an adjacent mammal tissue.

4. Medical device according to claim 1, selected from the group consisting of venflones; catheters, including urinary catheters, central vein catheters (CVC), peripheral vein catheters (PVC), and subcutaneous vein ports (SVP); drainage tubes, including tubes for pleura drainage and other wound or organ drainages; articles intended for supervision of pressure and/or blood gases; gaskets for colostomy bags; tubes for pharynx and trachea; and intravenous dressings.

5. Medical device according to claim 4, including said NO eluting polymer mixed with a core material, such as silicone, latex, polytetraflouroethene, polyurethane, polyvinylchloride, polycarbonate, Acrylonitrile Butadiene Styrene (ABS), polyacrylate, polyamide, polyethene, polypropene, polyolefins, polystyrene, rubbers, and/or any combinations of these.

6. Device according to claim 1, wherein said device is partly disintegrable when subjected to moisture or water.

7. Device according to any preceding claim, wherein said device comprises silver, configured for theraputic treatment of said mammal tissue.

8. Device according to any preceding claim, wherein said polymer is comprised in the device in form of fibers, nano-particles or micro-spheres.

9. Device according to claim 8, wherein said nano-particles, or micro-spheres, are integrated with, preferably encapsulated in, a material, selected from the group consisting of polyvinylacetates, polylacticacids, starch, cellulose, polyhydroxyalkanoates, polyesters, polycaprolactone, polyvinylalcohol, protein based plastics, and/or gelatine, or combinations thereof.

10. A manufacturing process for a intravascular, interstitial or intraorgan medical device configured according to any of claims 1-9, comprising:
selecting a plurality of nitric oxide eluting polymeric particles, preferably nano fibres, nano particles or micro spheres, and
deploying said nitric oxide eluting polymer in said medical device, or as a coating onto a core material of the device, to form said medical device,
wherein said deploying comprises electro, air, or gas stream spinning onto said core material, or mixing with said core material prior to the extrusion of said medical device.

11. Use of a nitric oxide (NO) eluting polymer for the manufacture of a intravascular, interstitial or intraorgan medical device according to any of claims 1-9,
wherein
nitric oxide is loaded to said device in such way that said device elutes nitric oxide (NO) from said eluting polymer in a therapeutic dose when used adjacent to mammal tissue.

12. Use according to claim 11, wherein said therapeutic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.
